# EUROPÄISCHE PATENTANMELDUNG

(11) **EP 3 112 865 A1**
(43) Veröffentlichungstag der Anmeldung: **04.01.2017**
(21) Anmeldenummer: 16001213.4
(22) Anmeldetag: 30.05.2016
(51) Int. Cl.: G01N 33/497

(54) **KALIBRIEREINRICHTUNG FÜR ATEMALKOHOLMESSGERÄTE**

(30) Priorität: 29.06.2015 DE 102015008303
(71) Anmelder: Dräger Safety AG & Co. KGaA, 23560 Lübeck (DE)
(72) Erfinder: Tschuncky, Peter, 23568 Lübeck (DE); Koch, Jochim, 23909 Ratzeburg (DE); Morley, Stefan, 23556 Lübeck (DE)
(74) Vertreter: Heinemeyer, Karsten

(57) **Zusammenfassung**

Dargestellt und beschrieben ist eine Kalibriereinrichtung (1) für ein Atemalkoholmessgerät (25) sowie ein System aus einer solchen Kalibriereinrichtung (1) und einem Atemalkoholmessgerät (25). Dabei ist in der Kalibriereinrichtung (1) ein Prüfgasbehälter (3) vorgesehen, der ein Prüfgas bereitstellt, das über einen Ausgang (5) in einen Einlass (11) eines Messadapters (9) strömen kann, wobei ein Messadapter (9) einen Strömungsweg (17) bildet, in dem das Prüfgas mit einem Staudruck beaufschlagt wird. Der Messadapter (9) ist zur Verbindung mit einem Atemalkoholmessgerät (25) ausgebildet, das eine Messung auslöst, wenn der Staudruck des Prüfgases einen Druckschweliwert des Atemalkoholmessgeräts (25) erreicht.

## Beschreibung

Die vorliegende Erfindung betrifft eine Kalibriereinrichtung für ein Atemalkoholmessgerät sowie ein System aus einer solchen Kalibriereinrichtung und einem Atemalkoholmessgerät.

Bei Atemalkoholmessgeräten ist es erforderlich, diese in regelmäßigen Abständen zu kalibrieren, um sicherzustellen, dass der angezeigte Messwert dem tatsächlichen Alkoholgehalt entspricht, der in der in das Gerät eingeblasenen Atemluft enthalten ist.

Zu diesem Zweck ist es hinlänglich bekannt, dass die fraglichen Geräte bei einer Serviceeinrichtung durch einen entsprechend geschulten Monteur neu kalibriert werden, wobei das Atemalkoholmessgerät einem Prüfgas ausgesetzt ist, das einen bekannten Alkoholgehalt hat. Ein solches, durch eine Serviceeinrichtung durchgeführtes Kalibrieren ist für den Benutzer außerordentlich aufwändig und daher nachteilhaft.

Darüber hinaus ist es aus der US 6,526,802 B1 bekannt, eine Wasser-Ethanol-Kalibrierflüssigkeit in einem geschlossenen Behälter zu verdampfen, mit dem das Atemalkoholmessgerät beaufschlagt wird, um dieses mit einem Gas mit einer definierten Alkoholkonzentration zu beschicken. Eine solche Vorrichtung hat sich jedoch auch als außerordentlich kompliziert in der Bedienung erwiesen.

Daher ist es ausgehend vom Stand der Technik die Aufgabe der vorliegenden Erfindung, eine Kalibriereinrichtung bereitzustellen, mit der ein Atemalkoholmessgerät in einfacher Weise kalibriert werden kann.

Gemäß einem ersten Aspekt der vorliegenden Erfindung wird diese Aufgabe durch eine Kalibriereinrichtung gelöst, mit einem Prüfgasbehälter, der ein unter einem Druck, der über dem Druck der die Kalibriereinrichtung umgebenden Atmosphäre liegt, stehendes Prüfgas an einem Ausgang bereitstellt, mit einem Messadapter, der einen Einlass und einen Auslass aufweist, wobei zwischen dem Einlass und dem Auslass ein Strömungsweg gebildet ist, wobei der Einlass mit dem Ausgang des Prüfgasbehälters verbunden ist, wobei der Auslass mit einem Strömungswiderstand versehen ist, wobei der Strömungswiderstand derart ausgestaltet ist, dass sich bei dem Prüfgas, das aus dem Ausgang des Prüfgasbehälters in den Einlass und durch den Auslass strömt, in dem Strömungsweg ein Staudruck aufbaut, der über einem vorgegebenen Wert liegt, und wobei der Messadapter an dem Strömungsweg einen Anschluss zur Verbindung mit einem Atemgaseinlass eines Atemalkoholmessgeräts aufweist.

Bei der erfindungsgemäßen Kalibriereinrichtung ist demnach ein Prüfgasbehälter mit einem unter Druck stehenden Prüfgas darin vorgesehen, wobei an den Ausgang des Prüfgasbehälters ein Messadapter angeschlossen ist, an dessen Auslass wiederum ein Strömungswiderstand vorgesehen ist, so dass dann, wenn Prüfgas aus dem Prüfgasbehälter durch den Messadapter strömt, sich in diesem ein Staudruck aufbaut. Das Prüfgas verdrängt dabei noch in dem Messadapter vorhandene Luft durch den Auslass, sodass diese Luft das Prüfgas nicht verunreinigt und den Alkoholgehalt des Prüfgases nicht beeinflusst. Der Strömungsweg ist außerdem mit einem Anschluss versehen, an den der Atemgaseinlass eines Alkoholmessgeräts angeschlossen werden kann. Wenn dieses der Fall ist und das Atemaikoholmessgerät aktiviert wird, startet es eine Messung, da der Staudruck in dem Messadapter derart gewählt ist, dass dessen Höhe mindestens dem Schwellwert entspricht, bei dem das Messgerät eine Messung startet, oder größer als dieser ist.

Durch einen solchen Aufbau wird sichergestellt, dass das Atemalkoholmessgerät mit einem Prüfgas mit einer definierten Alkoholkonzentration beaufschlagt wird, und es werden durch den Messadapter die Bedingungen simuliert, die bei einer realen Atemalkoholmessung eines Probanden gegeben sein müssen, d. h. ein Gasdruck mit vorgegebener Höhe in dem Atemgaseinlass des Messgeräts. Damit diese Bedingungen tatsächlich erfüllt sind, ist es lediglich herstellerseitig erforderlich, den Strömungswiderstand, der beispielsweise als eine Blende ausgebildet sein kann, entsprechend zu dimensionieren und Prüfgasbehälter mit einem erforderlichen Druck bereitzustellen.

Die Verwendung eines definierten Strömungswiderstandes ermöglicht einerseits, dass bei vorgegebenem Druck in dem Prüfgasbehälter sich in dem Strömungsweg ein vorgegebener Druck aufbaut aber andererseits auf komplizierte Vorrichtungen wie etwa Druckminderer verzichtet werden kann.

In einer bevorzugten Ausführungsform der Kalibriereinrichtung ist zwischen dem Einlass und dem Strömungsweg ein Ventil in dem Messadapter vorgesehen, so dass die Verbindung zwischen dem Messadapter bzw. dem darin vorgesehenen Strömungsweg und dem Prüfgasbehälter vom Benutzer geöffnet und geschlossen werden kann.

Darüber hinaus ist es bevorzugt, wenn an dem Einlass des Messadapters ein lösbarer Adapter vorgesehen ist, so dass der Messadapter an unterschiedliche Ausgänge unterschiedlicher Prüfgasbehälter angepasst werden kann.

Des Weiteren ist es bevorzugt, wenn es sich bei dem Prüfgas um ein mit Alkohol versetztes Trägergas handelt, und in besonders bevorzugter Weise handelt es sich um ein Ethanol-Luft-Gemisch.

Des Weiteren wird die obige Aufgabe durch ein System mit einem Atemalkoholmessgerät und einer zuvor beschriebenen Kalibriereinrichtung gelöst, wobei der Strömungswiderstand des Messadapters der Kalibriereinrichtung derart angepasst ist, dass der sich darin bildende Staudruck größer als der Schwellwert des Drucks ist, bei dem das Atemalkoholmessgerät eine Messung startet.

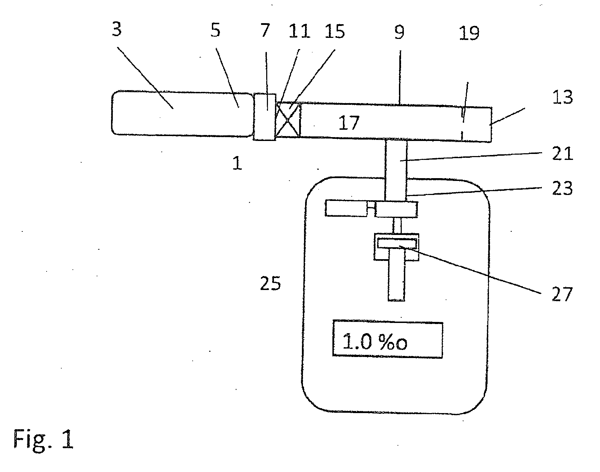

Im Folgenden wird die vorliegende Erfindung anhand einer Zeichnung erläutert, in der lediglich ein bevorzugtes Ausführungsbeispiel dargestellt ist, wobei
- Fig. 1: eine schematische Darstellung eines Ausführungsbeispiels eines Systems aus der Kalibriereinrichtung und einem Atemalkoholmessgerät zeigt.

Das Ausführungsbeispiel eines erfindungsgemäßen Systems umfasst eine Kalibriereinrichtung 1, die einen Prüfgasbehälter 3 aufweist, der mit einem Ausgang 5 versehen ist und in dem als Prüfgas ein Ethanol-Luft-Gemisch enthalten ist. Das Prüfgas steht dabei unter einem Druck, der über dem Druck der das System umgebenden Atmosphäre liegt.

Der Ausgang 5 des Prüfgasbehälters 3 ist über einen Adapter 7, der lösbar an einem Messadapter 9 angebracht ist, mit diesem verbunden, wobei der Messadapter 9 einen Einlass 11 und einen Auslass 13 aufweist. An dem Einlass 11 ist ein betätigbares Ventil 15 vorgesehen, das den Einlass 11 mit einem zwischen dem Einlass 11 und dem Auslass 13 gebildeten Strömungsweg 17 verbindet und durch einen Benutzer geöffnet werden kann.

Schließlich ist an dem Auslass 13 ein Strömungswiderstand 19 in Form einer Blende angeordnet, und an dem Strömungsweg 17 ist weiterhin ein Anschluss 21 angebracht, an den der Atemgaseinlass 23 des Atemalkoholmessgeräts 25 des Systems angeschlossen werden kann.

Der Strömungswiderstand 19 des Messadapters 9 ist derart dimensioniert, dass sich dann, wenn Prüfgas aus dem Ausgang 5 durch den Adapter 7 und das Ventil 15 weiter durch den Strömungsweg 17 aus dem Auslass 13 strömt, sich in dem Strömungsweg ein Staudruck aufbaut, der dem Druckschwellwert entspricht, bei dem das Atemalkoholmessgerät 25 eine Messung startet. Üblicherweise wird dazu durch den Hub einer Kolbenpumpe 27 in dem Atemalkoholmessgerät ein Teilvolumen eines unter einem Staudruck stehenden Atemgases aus dem Strömungsweg 17 entnommen und einem geeigneten Sensor zugeführt.

Um das Atemalkoholmessgerät 25 zu kalibrieren, wird die Kalibriereinrichtung 1 zunächst mit deren an dem Messadapter 9 vorgesehenen Anschluss 21 mit dem Atemgaseinlass 23 des Atemalkoholmessgeräts 25 verbunden. Anschließend wird das Ventil 15 geöffnet, so dass Prüfgas aus dem Prüfgasbehälter 3 durch den Strömungsweg 17 und den Strömungswiderstand 19 aus dem Auslass 13 ausströmt, wobei sich der schon erwähnte Staudruck in dem Strömungsweg 17 einstellt, Da dieser derart gewählt ist, dass er oberhalb der Druckschwelle liegt, bei der das Atemalkoholmessgerät 25 eine Messung startet, erfolgt eine solche Messung, wenn das Gerät 25 entsprechend aktiviert ist. Da das Prüfgas eine bekannte Alkoholkonzentration hat, kann eine solche Messung zur Kalibrierung des Geräts 25 verwendet werden. Damit ermöglicht die erfindungsgemäße Kalibriereinrichtung 1 bzw. das erfindungsgemäße System aus einer solchen Kalibriereinrichtung 1 und einem Atemalkoholmessgerät 25 eine einfache Kalibrierung des Messgeräts 25, die auch von einem Benutzer ohne große Fachkenntnisse ausgeführt werden kann.

## Patentansprüche

1. Kalibriereinrichtung (1) für Atemalkoholmessgeräte (25) mit einem Prüfgasbehälter (3), der ein unter einem Druck, der über dem Druck der die Kalibriereinrichtung umgebenden Atmosphäre liegt, stehendes Prüfgas an einem Ausgang (5) bereitstellt,
mit einem Messadapter (9), der einen Einlass (11) und einen Auslass (13) aufweist,
wobei zwischen dem Einlass (11) und dem Auslass (13) ein Strömungsweg (17) gebildet ist,
wobei der Einlass (11) mit dem Ausgang (5) des Prüfgasbehälters (3) verbunden ist,
wobei der Auslass (13) mit einem Strömungswiderstand (19) versehen ist,
wobei der Strömungswiderstand (19) derart ausgestaltet ist, dass sich bei dem Prüfgas, das aus dem Ausgang (5) des Prüfgasbehälters (3) in den Einlass (11) und durch den Auslass (13) strömt, in dem Strömungsweg (17) ein Staudruck aufbaut, der über einem vorgegebenen Wert liegt, und
wobei der Messadapter (9) an dem Strömungsweg (17) einen Anschluss (21) zur Verbindung mit einem Atemgaseinlass (23) eines Atemalkoholmessgeräts (25) aufweist.

2. Kalibriereinrichtung (1) nach Anspruch 1, wobei zwischen dem Einlass (11) und dem Strömungsweg (17) ein Ventil (15) vorgesehen ist, sodass die Verbindung mit dem Ausgang (5) des Prüfgasbehälters (3) geöffnet und geschlossen werden kann.

3. Kalibriereinrichtung (1) nach Anspruch 1 oder 2, wobei der Einlass (11) einen lösbaren Adapter (7) aufweist, der zwischen dem Einlass (11) und dem Ausgang (13) des Prüfgasbehälters (3) angeordnet ist.

4. Kalibriereinrichtung (1) nach einem der Ansprüche 1 bis3, wobei das Prüfgas ein Alkohol in einem Trägergas ist.

5. Kalibriereinrichtung (1) nach Anspruch 4, wobei das Prüfgas ein Ethanol-Luft-Gemisch ist.

6. System zur Atemalkoholmessung mit einem Atemalkoholmessgerät (25), das einen Atemgaseinlass (23) aufweist, durch den ein Proband Atemgas einblasen kann,
wobei das Messgerät (25) derart ausgestaltet ist, dass der Alkoholgehalt des Gases in dem Atemgaseinlass (23) dann bestimmt wird, wenn der Druck in dem Atemgaseinlass (23) einen vorgegebenen Schwellwert überschreitet, und
mit einer Kalibriereinrichtung (1) nach einem der Ansprüche 1 bis 5, die mit dem Atemgaseinlass (23) des Atemgasmessgeräts (25) verbunden werden kann, wobei der Staudruck derart gewählt ist, dass er größer als der vorgegebene Schwellwert ist.
